Europäisches Patentamt

European Patent Office

Office européen des brevets

(11) Publication number: **0 511 703 A2**

## EUROPEAN PATENT APPLICATION

(21) Application number: **92201103.6**

(22) Date of filing: **18.04.92**

(51) Int. Cl.5: **A61K 31/725**, //(A61K31/725, 31:70)

(30) Priority: **25.04.91 CH 1246/91**

(43) Date of publication of application:
**04.11.92 Bulletin 92/45**

(84) Designated Contracting States:
**AT BE DE DK ES FR GB GR IT LU MC NL PT SE**

(71) Applicant: **APR APPLIED PHARMA RESEARCH S.A.**
**Via Balestra, 27**
**CH-6901 Lugano(CH)**

(72) Inventor: **Vecchi, Giuseppe**
**Via Aldesago, 60**
**CH-6974 Aldesago(CH)**

(74) Representative: **Dragotti, Gianfranco et al**
**SAIC BREVETTI s.r.l. Viale Bianca Maria, 15**
**I-20122 Milano(IT)**

(54) **Pharmaceutical composition, containing sucralfate and carrageenan, useful for gastroenterology.**

(57) A composition for oral use containig as the active principle a combination of sucralfate and carrageenin is used in the therapy of diseases of the gastrointestinal tract such as ulcers and irritating and inflammatory states.

EP 0 511 703 A2

The present invention relates to the use in therapeutical field of a combination of sucralfate and carrageenin, as well as to compositions studied for the oral administration thereof which are particularly useful in the prevention and therapy of several diseases of the gastroenteric tract such as for example : gastric and duodenal ulcer, acute and chronical gastritis, gastroduodenitis, heart burn, esophageous relex, irritating and inflammatory states, originating from the administration of gastrolesive drugs, mechanical, thermal, chemical shocks etc.

Carrageenin is a polysaccharide present in some red algae of the genus Chondrus and Furcellaria, often found along the Atlantic coasts; it is chemically identified as a polymer of galactose and of 3,6-anhydro galactose containing a high number of salified sulfuric esters. Most commonly carrageenin is salified with the potassium ion and in such a form for example is widely used in the food field as thickening, suspending , gelifying and stabilizing agent.

The carrageenin owing to the presence of sulfuric groups distributed along the molecule, possesses interesting anti-inflammatory properties owing to which it is useful in the treatment of the gastric ulcers and of gastritis; moreover, thanks to the viscous protecting layer formed thereby onto the mucosae of the gastroenteric tract, has a useful protecting, pain removing and emollient action by which the irritations of whatever nature are attenuated (M. Pedretti, Chimica e Farmacobiologia delle piante medicinali, Studio Edizioni, 1990).

The sucralfate chemically is the aluminium basic salt of sulfuric octaester of saccharose, already known in therapeutical field as antiulcer cytoprotective drug (Merck Index, IIth edition).

The wide clinical documentation relating to sucralfate has demonstrated the effectiveness of this compound in the therapy of several gastrointestinal pathologies: gastric and duodenal ulcers, acute and chronical gastrities, gastroduodenitis, heartburn and esophageous reflex. It has already been demonstrated the effectiveness of the sucralfate in the prevention and the treatment of ulcers and also in the therapy looking to prevent the return of ulcerating lesions. The mechanism of action is mainly of mechanical type: it acts , as a matter of fact, locally by adhering in a selective manner to the areas of the gastrointestinal mucosae.

The sucralfate has shown the capability of influencing the synthesis and the endoluminal release of cytoprotective prostaglandins ($PGE_2$ and 6-keto-$PGF_1$a) as well as inhibiting the synthesis of $TXB_2$, so as to create a favourable effect on the microcirculation. In view of the action mechanism, the administration route which is used has always been the oral one. In the clinical studies several pharmaceutical forms have been used: tablets, granulates and suspensions (Brodgen R.N. et all. "Sucralfate. A review of its pharmacodynamic properties and therapeutic use in peptic ulcer disease". Drug. 27: 194-209 (1984)).

Taking still into consideration the action mechanism of the sucralfate the liquid pharmaceutical form in all heavier risks situations, with lower side effects, has been considered as preferable both as regards the effectiveness and as regards the tolerability. The liquid form as a matter of fact permits an increase of the contact surface between the drug and the area showing lesions; more over it has been found that the suspension is less liable to induce side effects and gastrointestinal reactions in comparison with granulate and tablets.

The combination of sucralfate and carrageenin, which is the object of the present invention, possesses peculiar therapeutical properties, such as for example a significant better protecting activity on the mucosae of the digesting tract in comparison with the single components; a possible explanation of this surprising therapeutical action can be related to a mechanism of synergic type. Also from the point of view of the physical behaviour , the combination according to the present invention possesses peculiar properties such as for example the capability of providing aqueous suspension characterized by a relevant stability to the decantation in the long run; these suspensions moreover possess a greater viscosity with respect to that shown under the same conditions by the single components (probably owing to an interaction between the active groups of the two molecules).

This feature causes the protecting effectivness on the gastroenteric mucosae to be sensibly improved; also the palatability is remarkably improved and it promotes the compliance of the patients and at a very end contributes to improve the effectivness of the therapy.

The pharmaceutical forms including the combination which is the object of the present invention are those known in the art of the pharmaceutical technique for the oral preparations.

Particularly and preferably, the combination according to the present invention is used in form of water suspensions, for the previously indicated reasons; this form leads to a more ready therapeutical action in comparison with the standard solid forms (tablets, capsules, etc.); more particularly in the esophageous reflex (which is a pathology widespread among old aged patients, and is particularly disturbing but also dangerous owing to the possibility of leading to even heavier complications) the aqueous suspensions containing the combination of the invention constitute a preferable drug owing to the protection which is obtained all along the esophagus tract .

The ratio between the sucralfate and carrageenin is not critical and varies within wide limits which can be advantageously exploited depending on the formulation which is desired; preferably, in the forms administered in solid state, the ratio must be of at least one part by weight of sucralfate for 0.1 parts by weight by carrageenin, in the liquid and semiliquid forms this ratio depends on the consistency degree which is desired in the suspension and anyhow for a suspension containing 10% by weight of sucralfate, percentages by weight of carrageenin of between 0.1 to 0.7% give place to liquid and semiliquid forms.

For example with a 10:1 ratio of sucralfate and carrageenin very dense suspensions are obtained; with a 3:1 ratio solid gelatins are practically obtained.

The posology of the combination is preferably referred to that which is known for sucralfate; on the average it is 2 to 10 g of sucralfate each day.

Hereinafter the compositions of some formulations are provided which are used with optimum results both from the palatability point of view and from the point of view of the real consistency and distribution.

EXAMPLE 1

Vial containing 200 ml of suspension having the following centesimal composition: 100 ml contain:

| | |
|---|---|
| - sucralfate | g 20 |
| - carrageenin | g 0.5 |
| - potassium sorbate | g 0.200 |
| - sorbitan (crystals) | g 12 |
| - aspartame | g 0.100 |
| - cream aroma | g 0.200 |
| - vanilla aroma | g 0.100 |
| - lemon aroma | g 0.300 |
| - polyvinylpyrrolidone | g 1 |
| - water | balance to 100 ml |

EXAMPLE 2

Thermowelded liquid envelopes containing 10 and 5 ml:

| | 10 ml | 5 ml |
|---|---|---|
| - sulcralfate | g 2 | g 1 |
| - carrageenin | g 0.005 | g 0.025 |
| - potassium sorbate | g 0.02 | g 0.01 |
| - sorbitan | g 12 | g 0.600 |
| - aspartame | g 0.01 | g 0.005 |
| - cream aroma | g 0.02 | g 0.01 |
| - vanilla aroma | g 0.001 | g 0.0015 |
| - lemon aroma | g 0.03 | g 0.05 |
| - polyvinylpyrrolidone | g 0.100 | g 0.05 |
| - water | balance to 10 ml enough to 5 ml | |

In any case, if all the above indicated components are unchanged apart from the amount of carrageenin, when the latter is increased from 2% to 10% by weight a constantly increasing consistency is obtained until the form of a jam results.

**Claims**

1.  Pharmaceutical composition characterized by containing as active ingredient the combination between sucralfate and carrageenin, together with the standard excipients and vehicles for pharmaceutical use.

2. Pharmaceutical composition according to claim 1, characterized in that the ratio between sucralfate and carrageenin in said combination is of at least one part by weight of sucralfate for 0.1 parts by weight of carrageenin.

3. Pharmaceutical composition according to claim 1, characterized in that it is in form suitable for the administration by oral route.

4. Pharmaceutical composition according to claim 3, characterized in that said oral form is a water suspension.

5. Pharmaceutical composition according to claim 4, characterized in that said suspension, for a content of 10% by weight of sucralfate, contains from 0.1 to 0.7% by weight of carrageenin.

6. Pharmaceutical composition according to claim 1, characterized by being indicated in the therapy of diseases of the gastroenteric tract, particularly gastric and duodenal ulcer, acute and chronical gastritis, gastroduodenitis, heartburn, esophageous relex, irritating and inflammatory states induced from gastrolesive drugs or from mechanical, thermal or chemical shocks.